# EUROPEAN PATENT SPECIFICATION

(11) **EP 2 638 198 B1**
(45) Date of publication and mention of the grant of the patent: **16.05.2018**
(21) Application number: 11794230.0
(22) Date of filing: 07.11.2011
(51) Int. Cl.: D06F 39/00, D06F 39/04

(54) **WASHING MACHINE FOR OBJECTS AND RELATIVE METHOD**
WASCHMASCHINE FÜR OBJEKTE UND ENTSPRECHENDES VERFAHREN
MACHINE À LAVER POUR OBJETS ET PROCÉDÉ ASSOCIÉ

(30) Priority: 08.11.2010 IT UD20100199
(43) Date of publication of application: 18.09.2013
(73) Proprietor: Steelco Spa, 31039 Riese Pio X (IT)
(72) Inventor: ZARDINI, Fabio, I-31033 Castelfranco Veneto (IT)
(74) Representative: Petraz, Gilberto Luigi
(86) International application number: PCT/IB2011/002623
(87) International publication number: WO 2012/063112

(56) References cited:
- EP-A1- 0 396 515
- EP-A1- 0 597 509
- EP-A2- 0 287 990
- EP-A2- 0 914 800
- WO-A1-2010/120657
- JP-A- 2008 082 565
- US-A- 3 789 860
- US-A- 4 326 551
- US-A- 4 580 421
- US-A- 5 301 701
- US-A- 5 660 193

## Description

### FIELD OF THE INVENTION

The present invention concerns a machine and washing method for objects, in particular but not restrictively, to wash and disinfect bulky objects, such as for example cages for laboratory animals.

### BACKGROUND OF THE INVENTION

Machines are known for washing bulky objects, such as for example cages for laboratory animals, comprising a washing chamber hydraulically connected to a washing unit and to a rinsing unit, each of which comprises a relative tank for containing the washing liquid and rinsing liquid. Furthermore, means to introduce chemical washing additives are generally also provided. Typically, the washing liquid is heated to a desired first temperature, for example about 50°C, chosen to optimize the action of the chemical additives.

Both units receive at entrance the relative liquids, generally water at a second temperature comprised between about 10°C and 15°C, from one or more external feeds, for example from the water network.

The rinsing liquid, fed at the second temperature, is heated to a third temperature, normally higher than the first temperature, for example about 80°C, suitable to determine a desired disinfection of the objects washed.

Generally the volumes of washing liquid and rinsing liquid are rather high, for example about 400 liters for the washing liquid and about 80 liters for the rinsing liquid, and therefore a considerable heat energy is required to take the liquids to the desired temperatures.

Once they have been used, the washing liquid and the rinsing liquid are normally discharged, completely dispersing their enthalpy content, which leads to an obvious waste of energy and time needed to re-heat the volumes of liquid in question, as well as the waste of liquid itself.

Document US-A-5,301,701 describes a plant for washing, rinsing and drying semi-conductor products.

The plant described in US'701 comprises a process chamber which is provided with a heating jacket, and a first and second tank for containing a heated cleaning solution which is fed inside the process chamber.

A third tank is also provided, for containing heated water which is introduced inside the jacket that surrounds the process chamber, in order to keep the latter at the desired temperature, or is introduced directly inside the process chamber for rinsing.

In a conventional manner, the cleaning solution is made to circulate in the process chamber and is then discharged beyond a certain level of contamination, while the heating water is made to circulate continuously in the jacket, thanks to a return pipe from the process chamber to the second tank, in order to keep the temperature of the process chamber constant, and the rinsing water is also discharged.

Purpose of the present invention is to achieve a machine and a relative method for washing objects that is economical in terms of energy costs and time.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a washing machine for objects, such as for example cages for laboratory animals or other bulky objects, comprises:
- a chamber in which the objects are washed and rinsed in sequence;
- a washing unit suitable to feed a washing liquid heated to a first temperature to the chamber;
- a feed circuit suitable to supply a rinsing liquid at a second temperature, lower than the first temperature;
- a rinsing unit suitable to feed the rinsing liquid received from the feed circuit heated to a third temperature, higher than the second temperature, advantageously also higher than the first temperature, to the chamber.

According to the present invention, the machine comprises means to recirculate the liquid used in the chamber at least toward the washing unit.

Moreover, heat exchange means are provided in cooperation between the washing unit and the rinsing unit so as to effect, directly or indirectly, a heat exchange between at least the liquid that is recirculated by the recirculation means and at least the rinsing liquid from the feed circuit that is fed to the rinsing unit.

According to the invention, the heat exchange means comprise first heat exchange means between the washing liquid of the first tank or arriving from the chamber, and the rinsing liquid arriving from the feed circuit, and second heat exchange means between the washing liquid arriving from the first tank and the rinsing liquid arriving from the feed circuit, disposed downstream of the first heat exchange means.

The washing machine has the advantage that, thanks to the recirculation means, it is possible to recover and re-use the liquid already used in the washing and rinsing chamber, thus preventing any waste of the liquids used. Furthermore, thanks to the heat exchange means in cooperation between the washing unit and the rinsing unit, it is possible to exchange heat energy between the washing unit and the rinsing unit and the associated feed circuit, so as to recover, thanks to the recirculation means, the heat energy of the liquids used in the chamber, before the liquids are discharged. The present invention therefore does not provide a simple recirculation of the pre-heated washing and rinsing liquids, as in the state of the art, but on the contrary provides a heat exchange between the two.

Therefore, the recirculation of the washing liquids and the sequential heat exchange between washing and rinsing liquids is useful for saving liquid and also for recovering the heat energy of the recirculation liquid.

Indeed, this energy would be lost if the liquid were discharged directly after the relative washing or rinsing step.

In some forms of embodiment, the washing unit comprises a first tank for the washing liquid, with which first heating means are associated, suitable to heat the washing liquid to the first temperature.

In some forms of embodiment, the first heat exchange means between the washing liquid and the rinsing liquid arriving from the feed circuit are disposed in the first tank.

According to other forms of embodiment, the washing unit comprises a first tub for a heat exchange liquid, advantageously the same liquid that is recirculated by the recirculation means, or a liquid introduced therein deliberately; the first tub is connected to the recirculation means upstream of the first tank. In these forms of embodiment, the first heat exchange means are disposed inside the first tub.

In variants of these forms of embodiment, the first tub is disposed in contact with the first tank, to promote the transmission of heat through conduction.

In some forms of embodiment, the machine comprises a heat exchange member hydraulically connected to the recirculation means, which is disposed through, through the washing unit.

In variants of these forms of embodiment, the heat exchange member is through in series through the first tub and the first tank, advantageously configured as a siphon, so as then to discharge at least part of the liquid recirculated.

In some forms of embodiment, the second heat exchange means between the washing liquid and the rinsing liquid arriving from the feed circuit, provided downstream of the first heat exchange means, are disposed in the first tank.

In some forms of embodiment, the rinsing unit comprises a second tank for the rinsing liquid, with which second heating means are associated, able to heat the rinsing liquid to the third temperature.

In some variants, the second heat exchange means are disposed in the second tank.

In some forms of embodiment, the rinsing unit comprises a second tub into which the liquid arriving from the feed circuit is introduced; the second tub is hydraulically connected to the second tank.

In some forms of embodiment, the second heat exchange means are disposed in the second tub.

In variant solutions, the second heat exchange means are configured to receive the washing liquid from the washing unit so as to exchange the heat of the washing liquid with the liquid fed by the feed circuit.

In variants of the present invention, the second tub is disposed in contact with the second tank to promote the transmission of heat through conduction.

The present invention also concerns a method for washing objects, comprising:
- a washing step in which a washing liquid, heated to a first temperature, is fed to a chamber in which the objects are washed and rinsed in sequence;
- a rinsing step in which a rinsing liquid is supplied at a second temperature, lower than the first temperature, in which the rinsing liquid is heated to a third temperature, advantageously higher than the first temperature, and in which the heated rinsing liquid is fed to the chamber.

According to one feature of the present invention, the method comprises a step of recirculating the liquid used in the chamber and a step of heat exchange, direct or indirect, between at least the liquid that is recirculated and at least the rinsing liquid that is supplied at the second temperature.

Furthermore, according to the present invention, the heat exchange step comprises a first sub-step of heat exchange between the rinsing liquid at the second temperature and the washing liquid, and a second heat exchange sub-step after the first sub-step, between the rinsing liquid before it is heated to the third temperature and the washing liquid

According to some variants, the washing step provides an accumulation of washing liquid in a first tank where it is heated to the first temperature.

According to variants of the method according to the invention, the first heat exchange sub-step occurs in the first tank.

In some forms of embodiment of the method, the washing step provides an accumulation of a heat exchange liquid in a first tub toward which the liquid used in the chamber is recirculated, the first heat exchange sub-step being carried out in the first tub.

In variant solutions, the method provides a heat exchange through conduction between the first tub and the first tank.

According to some forms of embodiment, the method provides a further heat exchange step, making the recirculated liquid pass through the washing liquid.

In variants of these forms of embodiment, the further heat exchange step occurs by passing the recirculated liquid in series through the first tub and the first tank.

According to some forms of embodiment, the second heat exchange sub-step, after the first sub-step, between the washing liquid and the rinsing liquid before it is heated to the third temperature, is carried out in the first tank.

According to some forms of embodiment of the method, the rinsing step provides an accumulation of the rinsing liquid in a second tank, where it is heated to the third temperature.

In some variant solutions, the second heat exchange sub-step is carried out in the second tank.

In further forms of embodiment, the rinsing step provides an accumulation of the rinsing liquid at the second temperature in a second tub, hydraulically connected to the second tank.

In variants of the invention, the second heat exchange sub-step is carried out in the second tub.

According to some forms of embodiment of the method, the second heat exchange sub-step provides to receives the washing liquid so as to exchange the heat of the washing liquid with the liquid fed for rinsing.

In other variants of the invention, the method provides a heat exchange through conduction between the second tub and the second tank.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a schematic representation of a washing machine;
- fig. 2 is a schematic representation of a washing step using the washing machine in fig. 1;
- fig. 3 is a schematic representation of another washing step using the washing machine in fig. 1;
- fig. 4 is a schematic representation of a rinsing step using the washing machine in fig. 1;
- fig. 5 is a schematic representation of another rinsing step using the washing machine in fig. 1.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF A PREFERENTIAL FORM OF EMBODIMENT

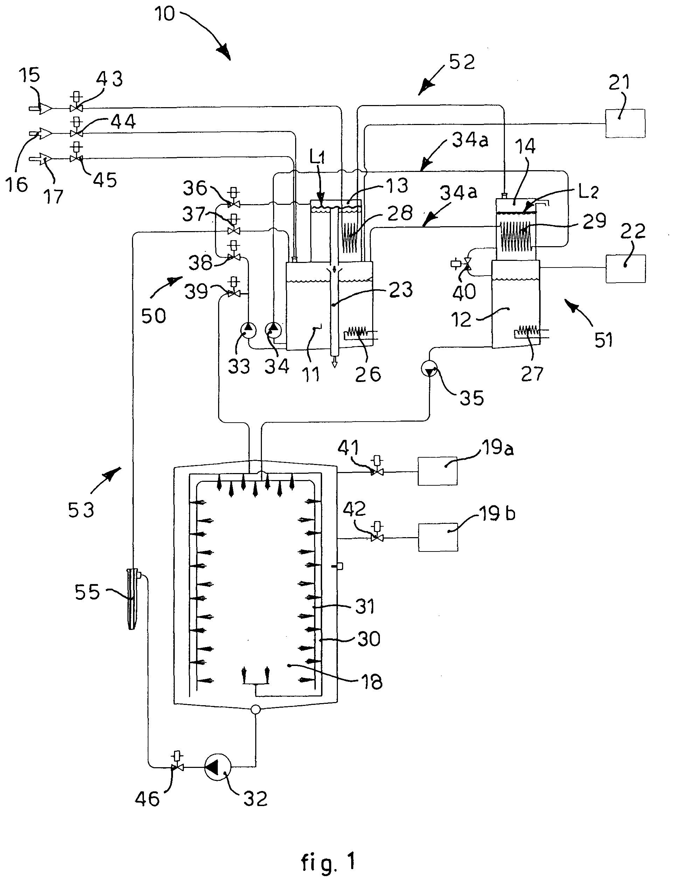

With reference to fig. 1, a washing machine 10 for objects according to the present invention is denoted in its entirety by the reference number 10, in particular for bulky objects such as for example cages for laboratory animals.

The washing machine 10 comprises a washing unit 50, a rinsing unit 51 and a washing chamber 18 of a size suitable to contain the objects.

Inside the washing chamber 18 there is a washing circuit 30 and a rinsing circuit 31 provided with delivery nozzles, respectively connected to the washing unit 50 and the rinsing unit 51. Means 19a, 19b are associated with the washing chamber 18 for drying the objects, cooperating by respective valves 41, 42, servo controlled.

The washing machine 10 is associated with feed lines 15, 16 and 17 to feed washing liquid and rinsing liquid, typically water from the water mains, at a given temperature, hereafter, to simplify the explanation, called second temperature, generally about 10°C-15°C, and in this case with introduction means 21, 22 to add chemical additives, respectively for washing and rinsing. The delivery rate of the feed lines 15, 16 and 17 is regulated by relative control valves 43, 44 and 45.

In particular, a feed circuit 52 for feeding the rinsing liquid takes the liquid from one of the feed lines 15, 16 and 17 and supplies it to the rinsing unit 51.

The washing unit 50 comprises a first tank 11 containing the washing liquid which, for example, has a capacity of about 400 liters.

The first tank 11 is provided with first heating means, for example a first electric resistance 26, to heat the washing liquid to a desired first temperature, typically about 45°C-55°C, for example 50°C, but in any case optimal for an effective action of the chemical washing additives.

The first tank 11 communicates with the hot and cold water feed lines 16 and 17, which supply the liquid that will be heated and, as explained hereafter, introduced into the washing chamber 18.

Furthermore, the first tank 11 is associated with a pump 33 and the valve means 39, which cooperate to allow to feed the liquid contained in the first tank 11 to the washing circuit 30 of the chamber 18, for washing purposes.

Service valve means 38 can possibly be provided in coordination with the valve means 37, by means of which to direct the liquid pumped by the pump 33 to the head of the first tank 11 instead of to the chamber 18.

In some forms of embodiment, the washing unit 50 also comprises a first tub, or accumulation tank 13, advantageously having a smaller volume than that of the first tank 11.

Advantageously, the first tub 13 is positioned in contact with the first tank 11, and in this way can exploit the phenomenon of direct heat conduction. In this case, the first tub 13 is positioned in contact with the first tank 11 above the latter, so as to also exploit the convective motions of the heat.

The machine 10 also comprises recirculation means, in this case a recirculation pipe 53, which develops from the exit of the chamber 18, from which it extracts the liquid used here, to the washing unit 50, and in particular can direct the recirculated liquid inside the first tub 13 or inside the first tank 11, selectively driving respective first and second valve means 36 and 37.

Furthermore, the washing unit 50 comprises a heat exchange member, in this case configured as a through pipe 23, which is in communication with the recirculation pipe 53, and develops from the first tub 13 through the first tank 11, passing through the liquid contained in the first tub 13 and the first tank 11, until it exits from the first tank 11 to discharge a desired quantity of recirculated liquid, advantageously at a reduced temperature, by virtue of the heat exchange performed, for example at about 35°C-40°C, reducing the environmental impact and heat impact on the discharge pipes associated with the machine, not shown here.

In particular, the through pipe 23 has an aperture or mouth, located in a zone near the top of the first tub 13 and which receives the excess liquid arriving from the first tub 13 when it is filled by the liquid recirculated by the recirculation pipe 53, and ends below and outside the first tank 11. The maximum level L1 of liquid in the first tub 13 is determined by the height at which the mouth of the through pipe 23 is located. When the level of liquid in the first tub 13 exceeds the maximum level LI, the excess liquid is discharged by the through pipe 23, which therefore functions as a siphon.

The rinsing unit 51 comprises a second tank 12 of rinsing liquid which, for example, has a capacity of about 80 liters.

The second tank 12, connected to the rinsing circuit 31 by means of a pump 35, is provided with second heating means, for example a second electric resistance 27, for heating the washing liquid to a desired third temperature, typically about 75°C-85°C, for example 80°C, in any case optimal for an at least partial action of disinfection during rinsing.

In some forms of embodiment, the rinsing unit 51 comprises a second tub or restoration tank 14, in hydraulic communication with the second tank 12, inside which the liquid arriving from the rinsing circuit 52 is fed.

Advantageously, the second tub 14 is positioned in contact with the second tank 12, in this case above the latter, and can thus exploit the phenomenon of direct heat conduction.

According to the present invention, heat exchange means are provided, by means of which to recover the heat of the recirculated liquid arriving from the chamber 18 to pre-heat the rinsing liquid supplied by the feed circuit 52.

In some forms of embodiment, the heat exchange means comprise a first exchanger 28 in line in the feed circuit 52, and disposed in the first tub 13, so as to exchange heat with the liquid contained therein. In some variants, not shown, the first exchanger 28 is disposed in the first tank 11.

In particular, a line 15 supplies the liquid, typically at 10°C-15°C, to the feed circuit 52, making it pass through the first exchanger 28. From here, the feed circuit 52 introduces the liquid, taken to a desired temperature, to the second tub 14.

The first exchanger 28 is sized to take the liquid, fed at a given rate, from the temperature of about 10°C-15°C to about 20°C-25°C.

Furthermore, the heat exchange means comprise a second exchanger 29 disposed in the second tub 14 downstream of the first exchanger 28. The liquid contained in the first tank 11 flows into the second exchanger 29, so as to exchange heat with the liquid contained in the second tub 14 and supplied by the feed circuit 52. In variants not shown, the second exchanger 29 is disposed in the first tank 11. In other variants not shown, the second exchanger 29 is disposed in the second tank 12.

In particular, a pump 34 is provided, associated with a circuit 34a that takes the liquid contained in the first tank 11 and, by means of a first branch of the circuit 34a, sends it to the second exchanger 29, from which, afterward, by means of a second branch of the circuit 34a, it is again introduced into the first tank 11.

The liquid in the second tub 14 is thus pre-heated to a desired temperature and, by means of selectively activated valve means 40, is introduced into the second tank 12. In particular, the second exchanger 29 is sized to take the liquid, already pre-heated thanks to the first exchanger 28, from the temperature of about 20°C-25°C to about 35°C-40°C.

There is therefore a quite obvious advantage in actuating a first heat exchange between the liquid in the first tub 13, which substantially functions as an accumulation of the heat energy of the washing liquid that is discharged, and the liquid arriving from the line 15, for a first increase in the temperature of the liquid intended for rinsing, and a second heat exchange between the liquid contained in the first tank 11 and the liquid arriving from the feed circuit 52, already subjected to a first heat exchange and accumulated in the second tub 14, for a second increase in the temperature of the liquid intended for rinsing. The liquid, once introduced into the second tank 12, is taken to the desired temperature by means of the second resistance 27, but starting from a higher temperature than that normally provided for the liquid fed by line 15. The saving in energy terms can be as much as 30%.

As mentioned above, the liquid used in the chamber 18, whether it is washing liquid or rinsing liquid, is recirculated to the washing unit 50 by means of the recirculation pipe 53 and with the aid of a pump 32 associated with valve means 46.

Downstream of the valve means 46 there is a filter 55 disposed in line in the recirculation pipe 53. The function of the filter 55 is to filter the recirculated liquid in order to remove the gross impurities present in the liquid at exit from the washing chamber 18.

In particular, by means of the valve means 36, able to be suitably activated, the recirculated liquid from the chamber 18 is introduced into the first tub 13 where, as explained above, it is introduced into the through pipe 23 and also the heat exchange with the first exchanger 28 occurs.

On the contrary, activating the second valve means 37 determines the introduction of the recirculated liquid directly into the first tank 11. This condition is normally adopted during the washing step, when the liquid is used several times in sequence to effect the washing in the chamber 18.

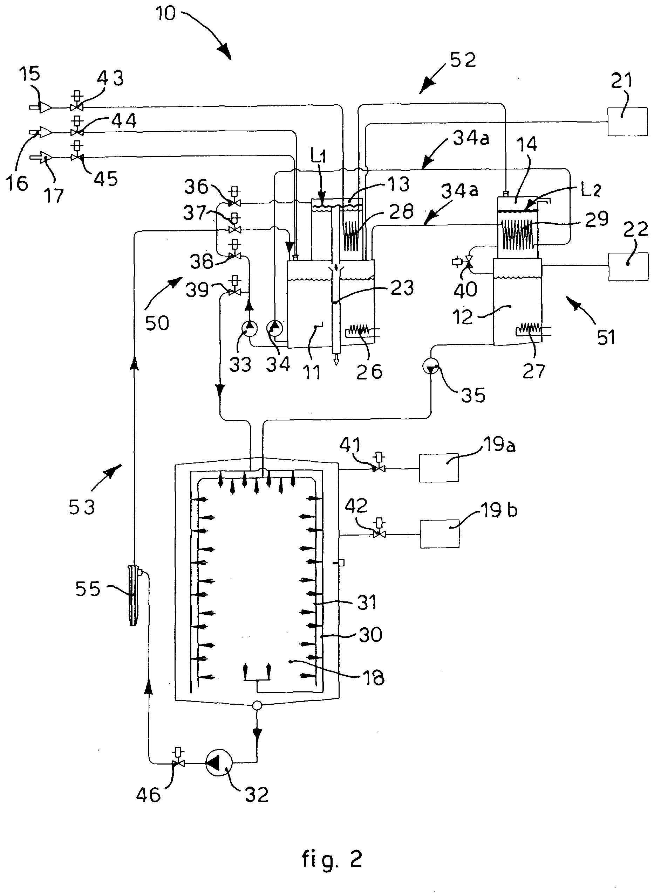

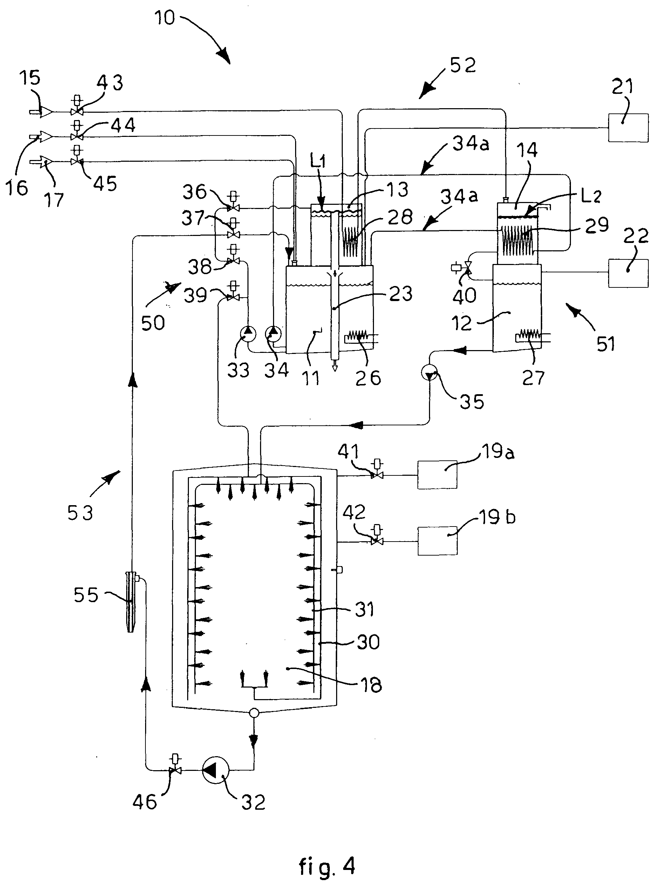

Figs. 2, 3, 4 and 5 show four steps in the washing process of the washing machine 10 for objects, showing by arrows the paths of the relative washing and rinsing liquids.

Fig. 2 shows a first step in the process during which the liquid contained in the first tank 11, taken to a first temperature suitable for the washing cycle by means of the resistance 26, in this case 50°C, is introduced thanks to the pump 33 and the valve means 39 into the washing circuit 30 inside the chamber 18 for washing the objects contained therein. In a synchronous manner, the chemical washing additives are added to the chamber 18 thanks to the introduction means 21.

The washing liquid used, collected on the bottom of the washing chamber 18, is then filtered and recirculated by means of the filter 55, the pump 33 that cooperates with the valve means 46 and activating in coordination the second valve means 37 in the first tank 11, where it can be re-used for one or more subsequent washing operations as above.

In this first step, therefore, washing occurs by continuously recirculating the volume of liquid, for example 400 liters of liquid, present in the first tank 11 and, during the first step, the recirculated liquid is kept at a temperature of about 50°C by the first resistance 26 inside the first tank 11.

When the washing step is terminated, there is a discharge step in which a part of the exhausted washing liquid contained in the first tank 11 is discharged.

As shown in fig. 2, during the discharge step the liquid used in the previous step and collected in the washing chamber 18 is filtered by the filter 55, and recirculated by the recirculation pipe 53, not inside the first tank 11 but rather, activating the first valve means 36 and de-activating in coordination the second valve means 37, it is introduced into the first tub 13.

As described above, the first tub 13 has a volumetric capacity lower than the first tank 11 and therefore accumulates the washing liquid until the maximum level L1 is reached. When the maximum level L1 has been reached, the excess part is discharged by means of the through pipe 23 which, as we said, is configured to function as a heat exchange member passing through the first tank 11. In the passage of the liquid through the through pipe 23, there is also a heat exchange with the liquid contained in the first tank 11, so as to recover a portion of heat energy that is given up to the washing liquid contained therein, at the same time obtaining the reduction of the temperature of the discharge to acceptable values, in particular in consideration of the environmental legislation currently in force. In particular, for a given flow rate, the through pipe 23 is sized so as to determine a heat exchange which reduces by about 10°C-15°C the temperature of the recirculated washing liquid before it is discharged.

In this case, assuming that the liquids arriving from the washing chamber 18 have a temperature of about 50°C, at exit from the through pipe 23 their temperature is reduced for example to about 40°C, without the aid of other cooling water. The discharge step, and simultaneous heat exchange, therefore allows to accumulate a part of the washing liquid in the first tub 13, so as to be able to exploit its heat energy at a later time thanks to the first exchanger 28, and to reduce the temperature of the discharge liquid exiting from the washing unit 50.

Furthermore, by providing the through pipe 53, it is possible during discharge to pre-heat the recovered liquid that is introduced into the first tank 11 through the feed lines 16 and 17.

When the discharge step is terminated, there is a third step in which the objects washed in the chamber 18 are rinsed. At the start of this third step, the second tank 12 of rinsing liquid contains for example about 80 liters of liquid which have been previously accumulated, thanks to the line 15 which supplies the rinsing liquid at a second temperature, about 10°C-15°C, to the feed circuit 52, and heated to a third temperature, for example about 80°C, by means of the second resistance 27.

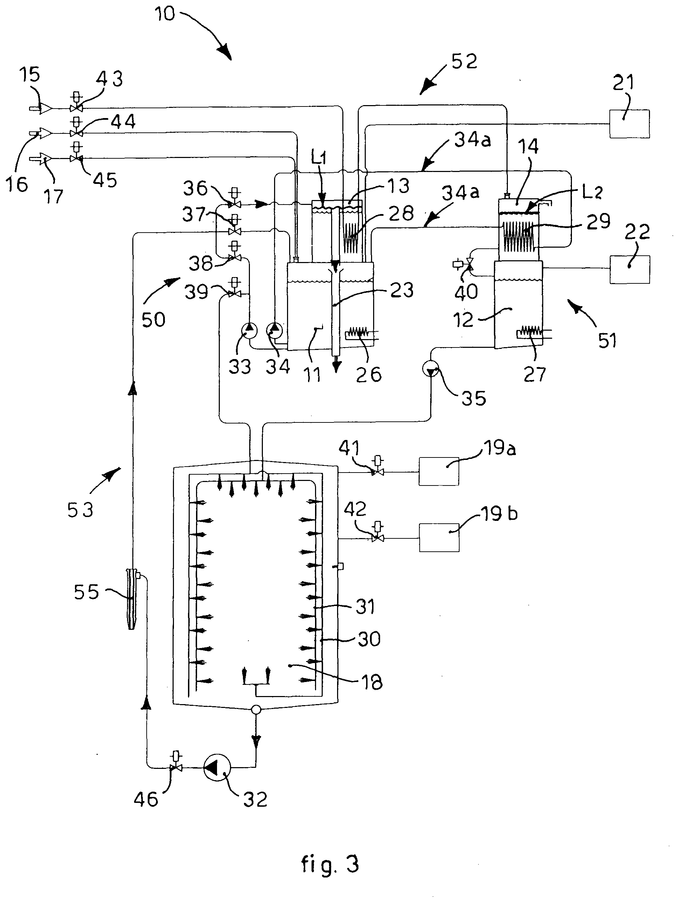

As shown in fig. 3, during the rinsing step, all the liquid contained in the second tank 12 is introduced thanks to the pump 35 into the rinsing circuit 31 of the chamber 18.

The rinsing liquid used and collected in the washing chamber 18 is generally not very contaminated and is therefore suitable to be re-used in a subsequent washing step. For this reason it is possible to recover the enthalpy content of this stream, introducing it into the first tank 11 by suitably activating the second valve means 37, obtaining a double advantage: recycling the liquid and re-using the heat accumulated in it, contributing to the rise in average temperature of the washing liquid in the first tank 11, giving benefits in terms of energy saving in the power required by the first resistance 26 to take the 400 liters of washing liquid to about 50°C. Another advantage of this solution is that, in the event of direct contact between the first tank 11 and the first tub 13, the heat accumulated in the first tank 11 is at least partly given up to the first tub 13 through conduction.

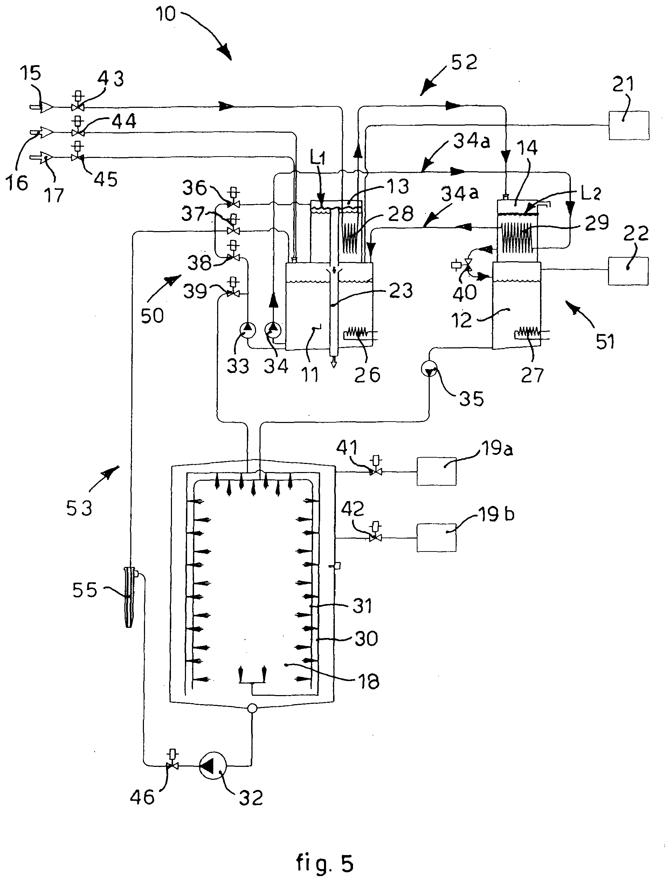

When the third rinsing step is terminated, there is a fourth step (fig. 5), in which the level L2 of the rinsing liquid in the second tank 12 is restored, using the liquid accumulated in the second tub 14. This liquid, supplied by line 15, is fed to the feed circuit 52 and first subjected to a double heat exchange in the first tub 13, thanks to the first exchanger 28, so as to obtain a first pre-heating thanks to the heat exchange with the recirculated washing liquid accumulated there, and in the second tub 14, thanks to the second exchanger 29, to obtain a second pre-heating thanks to the heat exchange with the washing liquid contained in the first tank 11, so as to save the heating power of the second resistance 27. In this case too, the heat conduction effect due to the direct contact between the second tub 14 and second tank 12 is advantageous. At the end of, or at the same time as, the recovery step, a new first washing step is begun.

## Claims

1. Washing machine for objects comprising:
- a chamber (18) in which the objects are washed and rinsed in sequence;
- a washing unit (50) suitable to feed a washing liquid heated to a first temperature to said chamber (18) and comprising a first tank (11) for the washing liquid, with which first heating means (26) are associated suitable to heat said washing liquid to said first temperature;
- a feed circuit (52) suitable to supply a rinsing liquid at a second temperature, lower than said first temperature;
- a rinsing unit (51) suitable to feed the rinsing liquid received from the feed circuit (52), heated to a third temperature, greater than the second temperature, advantageously greater than the first temperature too, to said chamber (18) and comprising a second tank (12) for the rinsing liquid, with which second heating means (27) are associated, suitable to heat said rinsing liquid to said third temperature;
**characterized in that** it comprises a recirculation pipe (53) configured to recirculate the liquid used in said chamber (18) from an exit at the bottom of the chamber (18) to at least said washing unit (50), the recirculation pipe (53) being connected to a pump (32) and to valve means (46), heat exchange means (28, 29) being provided in cooperation between the washing unit (50) and the rinsing unit (51) in order to carry out, directly or indirectly, a heat exchange between at least the liquid which is recirculated by the recirculation pipe (53) and at least the rinsing liquid which is fed to the rinsing unit (51) from the feed circuit (52), said heat exchange means comprising first heat exchange means (28) between the washing liquid of the first tank (11) or coming from the chamber (18) and the rinsing liquid coming from said feed circuit (52), and second heat exchange means (29) between the washing liquid coming from the first tank (11) and the rinsing liquid coming from said feed circuit (52), disposed downstream of said first heat exchange means (28), **in that** the washing unit (50) comprises a first tub (13) for a heat exchange liquid, said first tub (13) being connected to said recirculation pipe (53) upstream of said first tank (11) **and in that** said first heat exchange means (28) are disposed inside said first tub (13), the first tub (13) being disposed in contact with said first tank (11) in order to promote the transmission of heat by means of conduction.

2. Machine as in claim 1, **characterized in that** said first heat exchange means (28) are disposed in said first tank (11).

3. Machine as in claim 1, **characterized in that** it comprises a heat exchange member (23) hydraulically connected to said recirculation pipe (53), which is disposed through said washing unit (51), passing in series through the first tub (13) and the first tank (11) to then discharge at least part of the recirculated liquid.

4. Machine as in any claim hereinbefore, **characterized in that** said second heat exchange means (29) are disposed in said first tank (11).

5. Machine as in any claim from 1 to 3, **characterized in that** said second heat exchange means (29) are disposed in said second tank (12).

6. Machine as in claim 5, **characterized in that** the rinsing unit (51) comprises a second tub (14) in which the liquid coming from the feed circuit (52) is introduced, said second tub (14) being hydraulically connected to said second tank (12) **and in that** said second heat exchange means (29) are disposed in said second tub (14) and configured to receive the washing liquid from the washing unit (50) so as to exchange the heat of said washing liquid with the liquid fed from the feed circuit (52) which is present in the second tub (14).

7. Machine as in claim 6, **characterized in that** the second tub (14) is disposed in contact with said second tank (12) in order to promote the transmission of heat by means of conduction.

8. Method for washing objects by means of a washing machine as in any claims hereinbefore, comprising:
- a washing step in which a washing liquid, heated to a first temperature, is fed to a chamber (18) in which the objects are washed and rinsed in sequence and which provides an accumulation of the washing liquid in a first tank (11) where it is heated to said first temperature;
- a rinsing step in which a rinsing liquid is supplied at a second temperature, lower than said first temperature, in which the rinsing liquid is heated to a third temperature, advantageously higher than the first temperature, and in which the heated rinsing liquid is fed to said chamber (18) during the rinsing step and which provides an accumulation of the rinsing liquid in a second tank (12) where it is heated to said third temperature;
**characterized in that** it comprises a step of recirculating the liquid used in said chamber (18) and a direct or indirect heat exchange step, between at least the liquid which is recirculated from an exit at the bottom of the chamber (18) and at least the rinsing liquid which is supplied at the second temperature, **in that** said heat exchange step comprises a first heat exchange sub-step between the rinsing liquid at the second temperature and the washing liquid, and a second heat exchange sub-step, subsequent to the first sub-step, between the rinsing liquid before it is heated to the third temperature and the washing liquid, **in that** the washing step provides an accumulation of a heat exchange liquid in a first tub (13) to which the liquid used in said chamber (18) is recirculated, **and in that** said heat exchange sub-step is carried out in said first tub (13), a heat exchange by conduction being provided between the first tub (13) and said first tank (11).

9. Method as in claim 8 **characterized in that** said first heat exchange sub-step occurs in said first tank (11).

10. Method as in claim 8, **characterized in that** it provides a further heat exchange step between the recirculated liquid and the washing liquid, making the recirculated liquid pass in series through the first tub (13) and the first tank (11).

11. Method as in any claim from 8 to 10, **characterized in that** the second heat exchange sub-step is carried out in said first tank (11).

12. Method as in any claim from 8 to 10, **characterized in that** the second heat exchange sub-step is carried out in said second tank (12).

13. Method as in any claim from 8 to 10, **characterized in that** the rinsing step provides an accumulation of the rinsing liquid at the second temperature in a second tub (14) hydraulically connected to said second tank (12) **and in that** the second heat exchange sub-step is carried out in said second tub (14), providing to receive the washing liquid so as to exchange the heat of said washing liquid with the liquid fed for rinsing.

14. Method as in claim 13, **characterized in that** it provides heat exchange by conduction between the second tub (14) and said second tank (12).

## Patentansprüche

1. Waschmaschine für Gegenstände, die Folgendes umfasst:
- eine Kammer (18), in der die Gegenstände nacheinander gewaschen und abgespült werden;
- eine Wascheinheit (50), die zum Zuführen einer Waschflüssigkeit, die auf eine erste Temperatur erhitzt ist, zu der Kammer (18) geeignet ist und einen ersten Behälter (11) für die Waschflüssigkeit umfasst, mit dem erste Heizmittel (26) geeignet verbunden sind, um die Waschflüssigkeit auf die erste Temperatur zu erhitzen;
- eine Zuführleitung (52), die dazu geeignet ist, eine Abspülflüssigkeit bei einer zweiten Temperatur, die niedriger als die erste Temperatur ist, zuzuführen;
- eine Abspüleinheit (51), die dazu geeignet ist, die von der Zuführleitung (52) erhaltene Abspülflüssigkeit, die auf eine dritte Temperatur, höher als die zweite Temperatur, vorteilhafterweise auch höher als die erste Temperatur, erhitzt ist, der Kammer (18) zuzuführen und die einen zweiten Tank (12) für die Abspülflüssigkeit umfasst, mit dem zweite Heizmittel (27) geeignet verbunden sind, um die Abspülflüssigkeit auf die dritte Temperatur zu erhitzen;
**dadurch gekennzeichnet, dass** sie eine Rezirkulationsleitung (53) umfasst, die zum Rezirkulieren der in der Kammer (18) verwendeten Flüssigkeit von einem Ausgang am Boden der Kammer (18) zu mindestens der Wascheinheit (50) konfiguriert ist, wobei die Rezirkulationsleitung (53) mit einer Pumpe (32) und Ventilmitteln (46) verbunden ist, wobei Wärmeaustauschmittel (28, 29) zusammenwirkend zwischen der Wascheinheit (50) und der Abspüleinheit (51) vorgesehen sind, um direkt oder indirekt einen Wärmeaustausch zwischen zumindest der Flüssigkeit, die von der Rezirkulationsleitung (53) rezirkuliert wird, und zumindest der Abspülflüssigkeit, die der Abspüleinheit (51) von der Zuführleitung (52) zugeführt wird, auszuführen, wobei die Wärmeaustauschmittel erste Wärmeaustauschmittel (28) zwischen der Waschflüssigkeit des ersten Behälters (11) oder von der Kammer (18) kommend und der Abspülflüssigkeit, die von der Zuführleitung (52) kommt, und zweite Wärmeaustauschmittel (29) zwischen der von dem ersten Behälter (11) kommenden Waschflüssigkeit und der von der Zuführleitung (52) kommenden Abspülflüssigkeit, die stromabwärts des ersten Heizmittels (28) angeordnet sind, umfassen, **dadurch dass** die Wascheinheit (50) eine erste Wanne (13) für eine Wärmeaustauschflüssigkeit umfasst, wobei die erste Wanne (13) stromaufwärts mit der Rezirkulationsleitung (53) des ersten Behälters (11) verbunden ist, und **dadurch dass** die ersten Wärmeaustauschmittel (28) in der ersten Wanne (13) angeordnet sind, wobei die erste Wanne (13) in Kontakt mit dem ersten Behälter (11) angeordnet ist, um das Übertragen von Wärme mittels Leiten zu unterstützen.

2. Maschine nach Anspruch 1, **dadurch gekennzeichnet, dass** die ersten Wärmeaustauschmittel (28) in dem ersten Behälter (11) angeordnet sind.

3. Maschine nach Anspruch 1, **dadurch gekennzeichnet, dass** sie ein Wärmeaustauschelement (23) umfasst, das hydraulisch mit der Rezirkulationsleitung (53) verbunden ist, die durch die Wascheinheit (51) angeordnet ist und nacheinander durch die erste Wanne (13) und den ersten Behälter (11) hindurchläuft, um dann zumindest einen Teil der rezirkulierten Flüssigkeit abfließen zu lassen.

4. Maschine nach einem der vorhergehenden Ansprüche, **dadurch gekennzeichnet, dass** die zweiten Wärmeaustauschmittel (29) in dem ersten Behälter (11) angeordnet sind.

5. Maschine nach einem der Ansprüche 1 bis 3, **dadurch gekennzeichnet, dass** die zweiten Wärmeaustauschmittel (29) in dem zweiten Behälter (12) angeordnet sind.

6. Maschine nach Anspruch 5, **dadurch gekennzeichnet, dass** die Abspüleinheit (51) eine zweite Wanne (14) umfasst, in die die von der Zuführleitung (52) kommende Flüssigkeit eingeleitet wird, wobei die zweite Wanne (14) hydraulisch mit dem zweiten Behälter (12) verbunden ist, und **dadurch dass** die zweiten Wärmeaustauschmittel (29) in der zweiten Wanne (14) angeordnet und dazu konfiguriert sind, die Waschflüssigkeit von der Wascheinheit (50) aufzunehmen, um so die Wärme der Waschflüssigkeit mit der von der Zuführleitung (52) zugeführten Flüssigkeit, die in der zweiten Wanne (14) vorliegt, auszutauschen.

7. Maschine nach Anspruch 6, **dadurch gekennzeichnet, dass** die zweite Wanne (14) in Kontakt mit dem zweiten Behälter (12) angeordnet ist, um das Übertragen von Wärme mittels Leiten zu unterstützen.

8. Verfahren zum Waschen von Gegenständen mittels einer Waschmaschine nach einem der vorstehenden Ansprüche, das Folgendes umfasst:
- einen Waschschritt, bei dem eine auf eine erste Temperatur erhitzte Waschflüssigkeit einer Kammer (18) zugeführt wird, in der die Gegenstände nacheinander gewaschen und abgespült werden, und der eine Ansammlung der Waschflüssigkeit in einem ersten Behälter (11), wo sie auf die erste Temperatur erhitzt wird, bereitstellt;
- einen Abspülschritt, bei dem eine Abspülflüssigkeit bei einer zweiten Temperatur, die niedriger ist als die erste Temperatur, zugeführt wird, bei dem die Abspülflüssigkeit auf eine dritte Temperatur erhöht wird, die vorteilhafterweise höher als die erste Temperatur ist, und bei dem die erhitzte Abspülflüssigkeit während des Abspülschritts der Kammer (18) zugeführt wird, und der eine Ansammlung der Abspülflüssigkeit in einem zweiten Behälter (12), wo sie auf die dritte Temperatur erhitzt wird, bereitstellt;
**dadurch gekennzeichnet, dass** es einen Schritt des Rezirkulierens der in der Kammer (18) verwendeten Flüssigkeit und einen direkten oder indirekten Wärmeaustauschschritt zwischen zumindest der Flüssigkeit, die von einem Ausgang am Boden der Kammer (18) rezirkuliert wird, und zumindest der Abspülflüssigkeit, die bei der zweiten Temperatur zugeführt wird, umfasst, **dass** der Wärmeaustauschschritt einen ersten Wärmeaustauschunterschritt zwischen der Abspülflüssigkeit bei der zweiten Temperatur und der Waschflüssigkeit, und einen auf den ersten Unterschritt folgenden zweiten Wärmeaustauschunterschritt zwischen der Abspülflüssigkeit, bevor sie auf die dritte Temperatur erhitzt wird, und der Waschflüssigkeit umfasst, **dass** der Waschschritt eine Ansammlung einer Wärmeaustauschflüssigkeit in einer ersten Wanne (13) bereitstellt, zu der die in der Kammer (18) verwendete Flüssigkeit rezirkuliert wird, und **dass** der Wärmeaustauschunterschritt in der ersten Wanne (13) ausgeführt wird, wobei ein Wärmeaustausch durch Leiten zwischen der ersten Wanne (13) und dem ersten Behälter (11) bereitgestellt wird.

9. Verfahren nach Anspruch 8, **dadurch gekennzeichnet, dass** der erste Wärmeaustauschunterschritt im ersten Behälter (11) stattfindet.

10. Verfahren nach Anspruch 8, **dadurch gekennzeichnet, dass** es einen weiteren Wärmeaustauschschritt zwischen der rezirkulierten Flüssigkeit und der Waschflüssigkeit bereitstellt, bei dem die rezirkulierte Flüssigkeit nacheinander durch die erste Wanne (13) und den ersten Behälter (11) geleitet wird.

11. Verfahren nach einem der Ansprüche 8 bis 10, **dadurch gekennzeichnet, dass** der zweite Wärmeaustauschunterschritt in dem ersten Behälter (11) durchgeführt wird.

12. Verfahren nach einem der Ansprüche 8 bis 10, **dadurch gekennzeichnet, dass** der zweite Wärmeaustauschunterschritt in dem zweiten Behälter (12) durchgeführt wird.

13. Verfahren nach einem der Ansprüche 8 bis 10, **dadurch gekennzeichnet, dass** der Abspülschritt eine Ansammlung der Abspülflüssigkeit bei der zweiten Temperatur in einer zweiten Wanne (14) bereitstellt, die hydraulisch mit dem zweiten Behälter (12) verbunden ist, und dass der zweite Wärmeaustauschunterschritt in der zweiten Wanne (14) durchgeführt wird, die dafür bereitgestellt ist, die Waschflüssigkeit aufzunehmen, um so die Wärme der Waschflüssigkeit mit der zum Abspülen zugeführten Flüssigkeit auszutauschen.

14. Verfahren nach Anspruch 13, **dadurch gekennzeichnet, dass** es einen Wärmeaustausch durch Leiten zwischen der zweiten Wanne (14) und dem zweiten Behälter (12) bereitstellt.

## Revendications

1. Machine à laver pour objets, comprenant :
- une chambre (18) dans laquelle les objets sont lavés et rincés en séquence ;
- une unité de lavage (50) adaptée pour introduire un liquide de lavage chauffé à une première température dans ladite chambre (18), et comprenant un premier réservoir (11) pour le liquide de lavage, à laquelle des premiers moyens de chauffage (26) sont associés, adaptés pour chauffer ledit liquide de lavage à ladite première température ;
- un circuit d'alimentation (52) adapté pour acheminer un liquide de rinçage à une deuxième température, inférieure à ladite première température ;
- une unité de rinçage (51) adaptée pour introduire le liquide de rinçage reçu à partir du circuit d'alimentation (52), chauffé à une troisième température supérieure à la deuxième température, également avantageusement plus élevée que la première température, dans ladite chambre (18), et comprenant un deuxième réservoir (12) pour le liquide de rinçage, auquel des deuxièmes moyens de chauffage (27) sont associés, adaptés pour chauffer ledit liquide de rinçage à ladite troisième température ;
**caractérisée en ce qu'**elle comprend un tuyau de recirculation (53) conçu pour faire recirculer le liquide utilisé dans ladite chambre (18) depuis une sortie au fond de la chambre (18) à au moins ladite unité de lavage (50), le tuyau de recirculation (53) étant relié à une pompe (32) et à des moyens à soupape (46), des moyens d'échange de chaleur (28, 29) étant disposés en coopération entre l'unité de lavage (50) et l'unité de rinçage (51) afin d'effectuer, directement ou indirectement, un échange de chaleur entre au moins le liquide qui est amené à recirculer par le tuyau de recirculation (53) et au moins le liquide de rinçage amené à l'unité de rinçage (51) du circuit d'alimentation (52), lesdits moyens d'échange de chaleur comprenant des premiers moyens d'échange de chaleur (28) entre le liquide de lavage du premier réservoir (11) ou provenant de la chambre (18) et le liquide de rinçage provenant dudit circuit d'alimentation (52), et des deuxièmes moyens d'échange de chaleur (29) entre le liquide de lavage provenant du premier réservoir (11) et le liquide de rinçage provenant dudit circuit d'alimentation (52), disposés en aval desdits premiers moyens d'échange de chaleur (28), **en ce que** l'unité de lavage (50) comprend comprend une première cuve (13) pour un liquide d'échange de chaleur, ladite première cuve (13) étant relié audit tuyau de recirculation (53) en amont dudit premier réservoir (11), et **en ce que** lesdits premiers moyens d'échange de chaleur (28) sont disposés à l'intérieur de ladite première cuve (13), la première cuve (13) étant placée en contact avec ledit premier réservoir (11) afin de promouvoir la transmission de chaleur par conduction.

2. Machine selon la revendication 1, **caractérisée en ce que** lesdits premiers moyens d'échange de chaleur (28) sont placés dans ledit premier réservoir (11).

3. Machine selon la revendication 1, **caractérisée en ce qu'**elle comprend un élément d'échange de chaleur (23) raccordé hydrauliquement (53) audit tuyau de recirculation, qui est disposé à travers ladite unité de lavage (51), et passe en séquence par la première cuve (13) et le premier réservoir (11) pour décharger ensuite au moins une partie du liquide recirculé.

4. Machine selon n'importe laquelle des revendications précédentes, **caractérisée en ce que** lesdits deuxièmes moyens d'échange de chaleur (29) sont placés dans ledit premier réservoir (11).

5. Machine selon n'importe laquelle des revendications 1 à 3, **caractérisée en ce que** lesdits deuxièmes moyens d'échange de chaleur (29) sont placés dans ledit deuxième réservoir (12).

6. Machine selon la revendication 5, **caractérisée en ce que** l'unité de rinçage (51) comprend une deuxième cuve (14) dans laquelle est introduit le liquide provenant du circuit d'alimentation (52), ladite deuxième cuve (14) étant raccordée hydrauliquement audit deuxième réservoir (12) et **en ce que** lesdits deuxièmes moyens d'échange de chaleur (29) sont disposés dans ladite deuxième cuve (14) et sont conçus pour recevoir le liquide de lavage de l'unité de lavage (50), de manière à échanger la chaleur dudit liquide de lavage avec le liquide alimenté du circuit d'alimentation (52), présent dans la deuxième cuve (14).

7. Machine selon la revendication 6, **caractérisé en ce que** la deuxième cuve (14) est placée en contact avec ledit deuxième réservoir (12) afin de promouvoir la transmission de chaleur par conduction.

8. Procédé pour le lavage d'objets au moyen d'une machine à laver selon n'importe laquelle des revendications précédentes, comprenant
- une étape de lavage dans laquelle un liquide de lavage, chauffé à une première température, est alimenté à une chambre (18) dans laquelle les objets sont lavés et rincés en séquence, cette étape comportant une accumulation du liquide de lavage dans un premier réservoir (11) où il est chauffé à ladite première température ;
- une étape de rinçage dans laquelle un liquide de rinçage est alimenté à une deuxième température, inférieure à ladite première température, dans laquelle le liquide de rinçage est chauffé à une troisième température, avantageusement supérieure à la première température, et dans laquelle le liquide de rinçage chauffé est alimenté à ladite chambre (18) pendant l'étape de rinçage, cette étape comportant une accumulation du liquide de rinçage dans un deuxième réservoir (12) où il est chauffé à ladite troisième température ;
**caractérisé en ce qu'**il comprend une étape consistant de recirculation du liquide utilisé dans ladite chambre (18), et une étape d'échange de chaleur directe ou indirecte entre au moins le liquide recirculé d'une sortie au fond de la chambre (18) et au moins le liquide de rinçage alimenté à la deuxième température, **en ce que** ladite étape d'échange de chaleur comprend une première sous-étape d'échange de chaleur entre le liquide de rinçage à la deuxième température et le liquide de lavage, et une deuxième sous-étape d'échange de chaleur, suivant la première sous-étape, entre le liquides de rinçage avant qu'il ne soit chauffé à la troisième température et le liquide de lavage, **en ce que** l'étape de lavage comporte une accumulation d'un liquide d'échange de chaleur dans une première cuve (13) vers laquelle le liquide utilisé dans ladite chambre (18) est amené à recirculer, et **en ce que** ladite sous-étape d'échange de chaleur est effectuée dans ladite première cuve (13), un échange de chaleur par conduction étant réalisé entre la première cuve (13) et ledit premier réservoir (11).

9. Procédé selon la revendication 8, **caractérisé en ce que** ladite première sous-étape d'échange de chaleur a lieu dans ledit premier réservoir (11).

10. Procédé selon la revendication 8, **caractérisé en ce qu'**il comporte une autre étape d'échange de chaleur entre le liquide recirculé et le liquide de lavage, le liquide recirculé passant en séquence par la première cuve (13) et le premier réservoir (11).

11. Procédé selon n'importe laquelle des revendications 8 à 10, **caractérisé en ce que** ladite deuxième sous-étape d'échange de chaleur (29) est effectuée dans ledit premier réservoir (11).

12. Procédé selon n'importe laquelle des revendications 8 à 10, **caractérisé en ce que** ladite deuxième sous-étape d'échange de chaleur (29) est effectuée dans ledit deuxième réservoir (12).

13. Procédé selon n'importe laquelle des revendications 8 à 10, **caractérisé en ce que** l'étape de rinçage comporte une accumulation du liquide de rinçage à la deuxième température dans une seconde cuve (14) raccordée hydrauliquement audit deuxième réservoir (12) et **en ce que** la deuxième sous-étape d'échange de chaleur est effectuée dans ladite seconde cuve (14), ce qui permet de recevoir le liquide de lavage de manière à échanger de la chaleur dudit liquide de lavage avec le liquide alimenté pour le rinçage.

14. Procédé selon la revendication 13, **caractérisé en ce qu'**il comporte l'échange de chaleur par conduction entre la deuxième cuve (14) et ledit deuxième réservoir (12).
